# EUROPEAN PATENT APPLICATION

(11) **EP 4 389 074 A1**
(43) Date of publication of application: **26.06.2024**
(21) Application number: 22921647.8
(22) Date of filing: 01.12.2022
(51) Int. Cl.: A61F 2/24, A61M 25/00

(54) **DELIVERY CATHETER**

(30) Priority: 24.01.2022 CN 202210098907
(71) Applicant: Shanghai Trulive Medtech Co., Ltd., Shanghai 201206 (CN); Jiangsu Trulive Medtech Co., Ltd., Nantong, Jiangsu 226400 (CN)
(72) Inventor: SUN, Yantao, Shanghai 201206 (CN); WEI, Yongqiang, Shanghai 201206 (CN); ZHAO, Jing, Shanghai 201206 (CN)
(74) Representative: Nordmeyer, Philipp Werner
(86) International application number: PCT/CN2022/135763
(87) International publication number: WO 2023/138223

(57) **Abstract**

A delivery catheter, comprising a first catheter section (10), a second catheter section (20), a first pull wire assembly (30) and a second pull wire assembly (40); wherein the first pull wire assembly (30) comprises a first pull wire (32) and a first pull wire ring (31), when the first pull wire (32) is pulled, the first pull wire ring (31) drives the first catheter section (10) to bend; the second pull wire assembly (40) comprises a second pull wire (42) and a second pull wire ring (41), wherein the second pull wire ring (41) is disposed at a distal end of the second catheter section (20) and connected to a proximal end of the first catheter section (10); when the second pull wire (42) is pulled, the second pull wire ring (41) drives the second catheter section (20) to bend; an angle at which the first catheter section (10) can bend is greater than an angle at which the second catheter section (20) can bend. In this way, the delivery catheter has two bendable sections with different characteristics, such that the problem of little difference in terms of performance, such as bending angles, and having insufficient bending are solved.

## Description

### TECHNICAL FIELD

The present invention relates to the technical field of medical catheter and, in particular, to a delivery catheter.

### BACKGROUND

With the improvement of the quality of life and the advancement of medical standards, the average age of humans is increasing year by year, and the incidence of valvular heart disease is also increasing year by year. Due to the complexity of the human body, different types of valves are needed to treat valvular heart disease. Minimally invasive interventional surgery can satisfy patients who cannot tolerate thoracotomy. In different minimally invasive interventional surgeries, implants and delivery systems need to be accurately passed through different paths of blood vessels. Due to the limitations in structure and size of blood vessel, it is usually necessary to define delivery catheters thereon with different turning angles and turning radii.

Traditional delivery catheters adopt an antenna-like structure, made of plastic interlayers and metal winding wires similar to a sandwich structure. Turning is generally achieved by adjusting the winding density of the wire. In similar designs, it is generally to place a pull wire at a fixed point and use the pull wire to control the turning of the delivery catheter. This results in two problems: 1. There is not much difference in performance between the bendable section and the unbendable section. If the tension is not large enough, the curved section will not bend sufficiently; if the tension is too large, the unbendable section will bend under the action of the wire; 2. It is easy to cause a twist of the delivery catheter while the bendable section is bending under the action of the pull wire, resulting in insufficient bending and undesired bending direction.

Therefore, it is an urgent problem to develop a segmented bending structure that can distinguish different bending angles and a delivery catheter that can control the bending radius and bending angle during the delivery process.

### SUMMARY OF THE INVENTION

An object of the present invention is to provide a delivery catheter to solve the current problems of little difference in performance of the bendable section and insufficient bending of the bendable section.

In order to solve the above technical problems, the present invention provides a delivery catheter, comprising a first catheter section, a second catheter section, a first pull wire assembly and a second pull wire assembly; wherein the first pull wire assembly comprises a first pull wire and a first pull wire ring, wherein the first pull wire ring is disposed at a distal end of the first catheter section, the first pull wire is connected to the first pull wire ring and extends toward a proximal end of the first catheter section; when the first pull wire is pulled, the first pull wire ring drives the first catheter section to bend; the second pull wire assembly comprises a second pull wire and a second pull wire ring, wherein the second pull wire ring is disposed at a distal end of the second catheter section and connected to a proximal end of the first catheter section, the second pull wire is connected to the second pull wire ring and extends toward a proximal end of the second catheter section; when the second pull wire is pulled, the second pull wire ring drives the second catheter section to bend; an angle at which the first catheter section can bend is greater than an angle at which the second catheter section can bend.

Optionally, the first catheter section comprises a hypotube section, and the second catheter section comprises a serpentine section.

Optionally, the first catheter section comprises a bendable section and a transition section, and wherein the bendable section is connected to the transition section and is located distally to the transition section, and an angle at which the bendable section can bend is greater than an angle at which the transition section can bend.

Optionally, an end of the first pull wire ring is provided with a groove, and a portion of the first catheter section in connection with the first pull wire ring is provided with a protrusion, or an end of the first pull wire ring is provided with a protrusion, and a portion of the first catheter section in connection with the first pull wire ring is provided with a groove, wherein the first wire pull ring and a distal end of the first catheter section are connected through matching of the groove and the protrusion; or an end of the second pull wire ring is provided with a groove, and a portion of the second catheter section in connection with the second pull wire ring is provided with a protrusion, or an end of the second pull wire ring is provided with a protrusion, and a portion of the second catheter section in connection with the second pull wire ring is provided with a groove, wherein the second wire pull ring and a distal end of the second catheter section are connected through matching of the groove and the protrusion; or an end of the second pull wire ring is provided with a groove, and a portion of the first catheter section in connection with the second pull wire ring is provided with a protrusion, or an end of the second pull wire ring is provided with a protrusion, and a portion of the first catheter section in connection with the second pull wire ring is provided with a groove, wherein the second wire pull ring and a proximal end of the first catheter section are connected through matching of the groove and the protrusion.

Optionally, a connection between the first pull wire ring and the first catheter section, a connection between the second pull wire ring and the first catheter section, or a connection between the second wire pull ring and the second catheter section, is formed by matching exclusive connection structures; or an exclusive connection structure is provided on the first pull wire ring along a circumferential direction thereof to connect to the first catheter section, and a further exclusive connection structure is provided on the second pull wire along a circumferential direction thereof to connected to the first catheter section or the second catheter section.

Optionally, a positioning mark is provided at the proximal end or the distal end of the first catheter section, and the positioning mark is configured to determine a circumferential position of the first catheter section.

Optionally, the first pull wire ring or the second pull wire ring has a pull wire clamping groove in which the first pull wire or the second pull wire can be clamped.

Optionally, the delivery catheter further comprises a lining tube disposed inside the first catheter section and the second catheter section, and the first pull wire is disposed between the first catheter section, the second catheter section and the lining tube, and the second pull wire is disposed between the second catheter section and the lining tube.

Optionally, a body of the first pull wire ring or the second pull wire ring has a hole into which the lining tube can expand outward so that a friction resistance between the lining tube and the first pull wire ring or the second pull wire ring is increased.

Optionally, the delivery catheter further comprises a covering tube disposed outside the first catheter section and the second catheter section.

In a delivery catheter provided by the present invention, the delivery catheter comprises a first catheter section, a second catheter section, a first pull wire assembly and a second pull wire assembly; wherein the first pull wire assembly comprises a first pull wire and a first pull wire ring, wherein the first pull wire ring is disposed at a distal end of the first catheter section, the first pull wire is connected to the first pull wire ring and extends toward a proximal end of the first catheter section; when the first pull wire is pulled, the first pull wire ring drives the first catheter section to bend; the second pull wire assembly comprises a second pull wire and a second pull wire ring, wherein the second pull wire ring is disposed at a distal end of the second catheter section and connected to a proximal end of the first catheter section, the second pull wire is connected to the second pull wire ring and extends toward a proximal end of the second catheter section; when the second pull wire is pulled, the second pull wire ring drives the second catheter section to bend; an angle at which the first catheter section can bend is greater than an angle at which the second catheter section can bend. With this arrangement, the delivery catheter can have two bendable sections with different characteristics. The first pull wire assembly is configured to control the bending of the first catheter section, and the second pull wire assembly is configured to control the bending of the second catheter section, so that the bending of the first catheter section and the second catheter section is controllable. It can realize a segmented bending structure with different bending angles, achieving different bending radii, bending angles and bending directions of the catheter during delivery. It can also realize a multipoint control of bending, which facilitates the implant to pass through the blood vessel smoothly and be delivered to the target location, and thus the problems of little difference in terms of the performance of the bendable section, such as bending angles, and having insufficient bending are solved.

### BRIEF DESCRIPTION OF THE DRAWINGS

Those of ordinary skill in the art will understand that the drawings are provided for a better understanding of the invention and do not constitute any limitation on the scope of the invention.
Fig. 1 is a schematic diagram of a delivery catheter according to an embodiment of the present invention;
Fig. 2 is a side view of a second catheter section of the delivery catheter according to an embodiment of the present invention;
Fig. 3 is a schematic diagram of a first pull wire ring according to an embodiment of the present invention;
Fig. 4 is a schematic diagram of a second pull wire ring according to an embodiment of the present invention;
Fig. 5 is a schematic diagram of a first catheter section according to an embodiment of the present invention;
Fig. 6 is a schematic diagram of a second catheter section according to an embodiment of the present invention;
Fig. 7 is a schematic diagram showing the bending of the first catheter section of the delivery catheter according to an embodiment of the present invention;
Fig. 8 is a schematic diagram showing the bending of the second catheter section of the delivery catheter according to an embodiment of the present invention.

In the drawings:
10, first catheter section; 11, bendable section; 12, transition section; 13, positioning mark; 101, first catheter section protrusion; 102, first catheter section groove;
20, second catheter section; 21, rotating ring; 22, bending notch; 201, second catheter section protrusion;
30, first pull wire assembly; 31, first pull wire ring; 311, first pull wire clamping groove; 312, first pull wire ring groove; 313, round hole; 32, first pull wire;
40, second pull wire assembly; 41, second pull wire ring; 411, second pull wire clamping groove; 412, second pull wire ring protrusion; 413, second pull wire ring groove; 42, second pull wire;
50, lining tube;
60, pull wire cavity;
70, covering tube;
80, braided wire;
1, first bending;
2, second bending.

### DETAILED DESCRIPTION

To make the objectives, advantages and features of the present invention clearer, the present invention will be further described in detail below with reference to the figures and embodiments. It is noted that the figures are provided in a very simplified form not necessarily presented to scale, with their only intention to facilitate convenience and clarity in explaining the disclosed embodiments. In addition, the structures shown in the figures are often part of the actual structure. In particular, the figures generally give emphasis on different details and are accordingly drawn to different scales.

As used herein, the singular forms of "a", "an" and "the" include plural references unless the context clearly dictates otherwise. As used herein, the term "or" refers to "and/or" unless the context clearly dictates otherwise. The term "several" is generally used in the sense of including "at least one", and the term "at least two" is generally used in the sense of including "two or more". In addition, the term "first", "second" and "third" are used for descriptive purposes only and cannot be understood as indicating or implying relative importance or implicitly indicating the quantity of indicated technical features. Therefore, the features defined as "first", "second" and "third" may explicitly or implicitly include one or at least two of these features, and the terms "mount", "couple" and "connect" shall be broadly understood, for example, it can be a fixed connection, a detachable connection, or an integral body; it can be a direct connection or an indirect connection through an intermediate medium; it can be an internal connection between two elements or an interaction between two elements. In addition, as used in the present invention, one element is disposed on another element, which usually only means that there is a connection, coupling, matching or transmission relationship between the two elements, and the relationship between the two elements may be direct or indirect through an intermediate element. This cannot be understood as indicating or implying the spatial positional relationship between two elements, that is, one element can be in any position inside, outside, above, below or to one side of another element, unless the content clearly stated otherwise. For those of ordinary skill in the art, the specific meanings of the above terms in the present invention can be understood according to specific circumstances. Additionally, in the following description, numerous specific details are given in order to provide a more thorough understanding of the invention. However, it will be apparent to one skilled in the art that the present invention may be practiced without one or more of these details. In other examples, some technical features that are well known in the art are not described in order to avoid confusion with the present invention.

An embodiment of the present invention provides a delivery catheter. The delivery catheter includes: a first catheter section, a second catheter section, a first pull wire assembly, and a second pull wire assembly. The first pull wire assembly includes a first pull wire and a first pull wire ring. The first pull wire ring is disposed at the distal end of the first catheter section. The first pull wire is connected to the first pull wire ring and extends toward the proximal end of the first catheter section. When the first pull wire is pulled, the first pull wire ring drives the first catheter section to bend. The second pull wire assembly includes a second pull wire and a second pull wire ring. The second pull wire ring is disposed at the distal end of the second catheter section, and is connected to the proximal end of the first catheter section. The second pull wire is connected to the second pull wire ring, and extends toward the proximal end of the second catheter section. When the second pull wire is pulled, the second pull wire ring drives the second catheter section to bend. The bending angle of the first catheter section is greater than the bending angle of the second catheter section. With this arrangement, the delivery catheter can have two bendable sections with different characteristics. The first pull wire assembly is configured to control the bending of the first catheter section, and the second pull wire assembly is configured to control the bending of the second catheter section, so that the bending of the first catheter section and the second catheter section is controllable. It can realize a segmented bending structure with different bending angles, achieving different bending radii, bending angles and bending directions of the catheter during delivery. It can also realize a multipoint control of bending, which facilitates the implant to pass through the blood vessel smoothly and be delivered to the target location, and thus the problems of little difference in terms of the performance of the bendable section, such as bending angles, and having insufficient bending are solved.

Description will be made below with reference to the drawings.

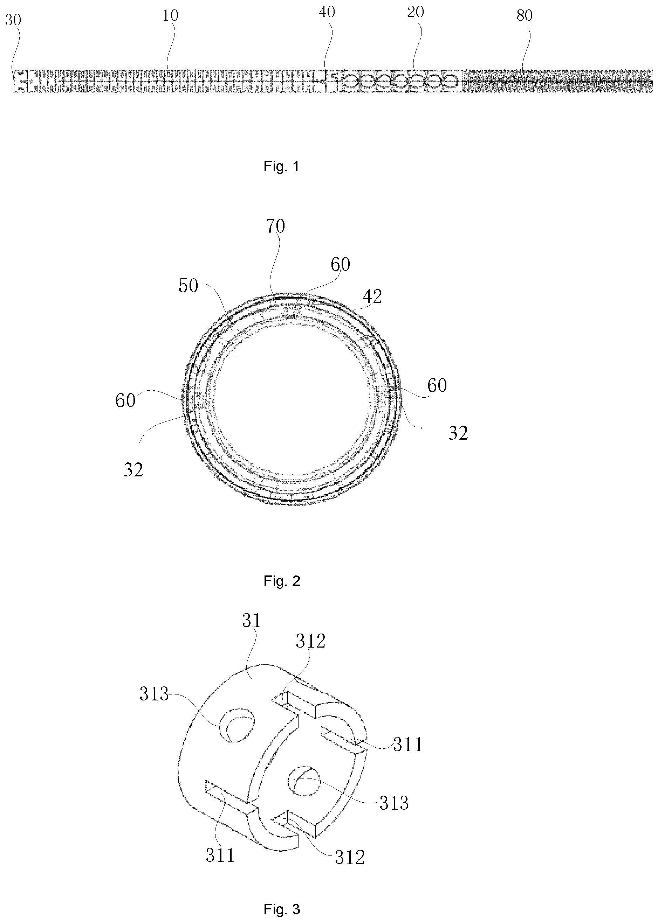

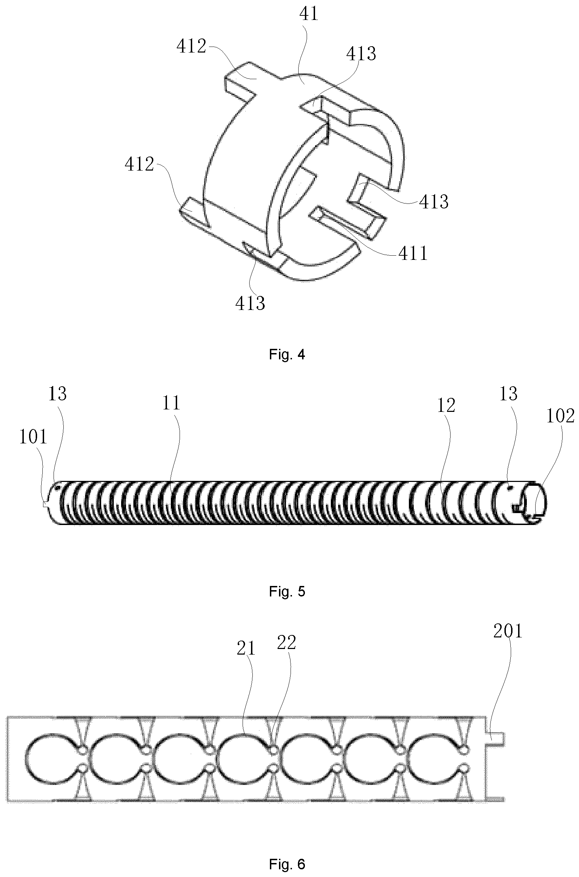

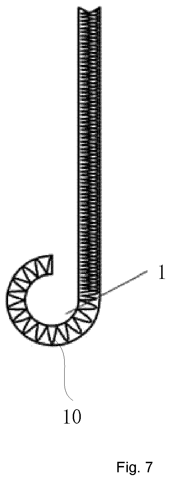

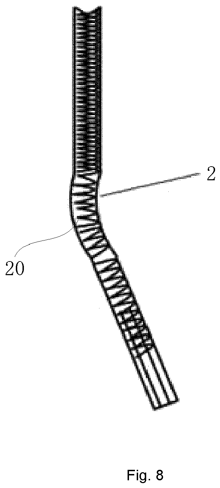

Please refer to Figs. 1 to 8, Fig. 1 is a schematic diagram of a delivery catheter according to an embodiment of the present invention; Fig. 2 is a side view of a second catheter section of the delivery catheter according to an embodiment of the present invention; Fig. 3 is a schematic diagram of a first pull wire ring according to an embodiment of the present invention; Fig. 4 is a schematic diagram of a second pull wire ring according to an embodiment of the present invention; Fig. 5 is a schematic diagram of a first catheter section according to an embodiment of the present invention; Fig. 6 is a schematic diagram of a second catheter section according to an embodiment of the present invention; Fig. 7 is a schematic diagram showing the bending of the first catheter section of the delivery catheter according to an embodiment of the present invention; Fig. 8 is a schematic diagram showing the bending of the second catheter section of the delivery catheter according to an embodiment of the present invention.

As shown in Fig. 1, this embodiment provides a delivery catheter. The delivery catheter is mainly used for delivery of implants in blood vessels. The delivery catheter includes: a first catheter section 10, a second catheter section 20, a first pull wire assembly 30 and a second pull wire assembly 40.

The first pull wire assembly 30 includes a first pull wire 32 and a first pull wire ring 31. The first pull wire ring 31 is disposed at the distal end of the first catheter section 10. The first pull wire 32 is connected to the first pull wire ring 31 and extends toward the proximal end of the first catheter section 10. When the first pull wire 32 is pulled, the first pull wire ring 31 drives the first catheter section 10 to bend. Specifically, the first catheter section 10 is, for example, a bendable tube. The first pull wire 32 preferably passes through the first catheter section 10 from the proximal end thereof and is connected to the first pull wire ring 31 at the distal end of the first catheter section 10. The distal end of the first pull wire 32 is connected to the first pull wire ring 31. The proximal end of the first pull wire 32 is located at the proximal side of the first catheter section 10. When the proximal end of the first pull wire 32 is pulled, the first pull wire 32 pulls the first pull wire ring 31 to move sideward, thereby driving the first catheter section 10 to bend. The first pull wire 32 may be located inside the first catheter section 10 or outside the first catheter section 10.

The second pull wire assembly 40 includes a second pull wire 42 and a second pull wire ring 41. The second pull wire ring 41 is disposed at the distal end of the second catheter section 20 and connected to the proximal end of the first catheter section 10. The second pull wire 42 is connected to the second pull wire ring 41 and extends toward the proximal end of the second catheter section 20. When the second pull wire 42 is pulled, the second pull wire ring 41 drives the second catheter section to bend. Similarly, the second catheter section 20 is, for example, a bendable tube. The second pull wire 42 preferably passes through the second catheter section 20 from the proximal end thereof and is connected to the second pull wire ring 41 at the distal end of the second catheter section 20. The distal end of the second pull wire 42 is connected to the second pull wire ring 41. The proximal end of the second pull wire 42 is located on the proximal side of the second catheter section 20. When the proximal end of the second pull wire 42 is pulled, the second pull wire 42 pulls the second pull wire ring 41 to move sideward, thereby driving the second catheter section 20 to bend. Similarly, the second pull wire 42 may be located inside the second catheter section 20 or outside the second catheter section 20.

The angle at which the first catheter section 10 can bend is greater than the angle at which the second catheter section 20 can bend. For example, as shown in Fig. 7, the distal end of the first catheter section 10 is bent under the action of the first pull wire 32, and the bending angle is preferably between 0° and 270°. As shown in Fig. 8, the distal end of the second catheter section 20 is bent under the action of the second pull wire 42, and the bending angle is preferably between 0° and 60°. In other embodiments, the angle at which the first catheter section 10 can bend may be greater than the angle at which the second catheter section 20 can bend. With this arrangement, the delivery catheter can have two bendable sections with different characteristics. Therefore, the bending of the first catheter section and the second catheter section is controllable. It can realize a segmented bending structure with different bending angles, achieving different bending radii, bending angles and bending directions of the catheter during delivery. It can also realize a multipoint control of bending, which facilitates the implant to pass through the blood vessel smoothly and is delivered to the target location, and thus the problems of little difference in terms of the performance of the bendable section, such as bending angles, and having insufficient bending are solved.

Preferably, as shown in Fig. 1, the first catheter section 10 includes a hypotube section, and the second catheter section 20 includes a serpentine section. The angle at which the hypotube section can bend is greater than the angle at which the serpentine catheter section can bend. The hypotube section enables crimping of the distal end of the delivery catheter. The serpentine section can avoid a large contraction of the delivery catheter in the axial direction and can achieve a large change in the rotation angle. In this way, the delivery catheter has different types of catheter sections, thereby realizing that the delivery catheter has a segmented bending structure with different bending angles, improving the delivery efficiency of the delivery catheter. The hypotube section or the serpentine section can be prepared by, but is not limited to, laser cutting, machining, pouring and lost-wax sintering. The serpentine section of the second catheter section 20 includes a rotating ring 21 and a bending notch 22. The rotating ring 21 allows the serpentine section to move around it. The bending notch 22 allows the serpentine section to move in the direction of the bending notch. The rotating ring 21 and the bending notch 22 work together to make the serpentine section to move left and right along its axis instead of up and down. In this embodiment, the bending angle and bending radius of the hypotube section and the serpentine section depend on the compactness of the serpentine section and the length of the bending notch of the hypotube section. For example, the less compactness the serpentine section, the larger the bending angle can be. The bending notch of the hypotube section becomes larger, the bending angle becomes larger, and the bending radius becomes smaller. By using hypotube section and serpentine section together, the bending control of different sections without mutual restriction is achieved.

Preferably, as shown in Fig. 5, the first catheter section 10 includes a bendable section 11 and a transition section 12. The bendable section 11 is connected to the transition section 12 and is located distally to the transition section 12. The angle at which the bendable section 11 can bend is greater than the angle at which the transition section 12 can bend, thereby maximizing the bending angle of the distal end of the delivery catheter. The angle at which the transition section 12 can bend ranges between that of the bendable section 11 and the second catheter section 20. Therefore, the bending performance of the delivery catheter is further improved and the delivery efficiency is improved. For example, the first catheter section 10 includes a hypotube section. The bendable section 11 of the hypotube section is greater in compactness than the transition section 12 of the hypotube section. Therefore, the bendability is better, and small radius and wide-angle bending can be achieved. In addition, the provision of the transition section 12 can also prevent the first catheter section 10 from breaking during the bending process.

Preferably, as shown in Fig. 3, an end of the first pull wire ring 31 is provided with a groove/protrusion, and the portion of the first catheter section 10 in connection to the first pull wire ring 31 is provided with a protrusion/groove. The first wire pull ring 31 and the distal end of the first catheter section 10 are connected through the matching of the groove and the protrusion, so that the first wire pull ring assembly and the first catheter section 10 can be connected well. Preferably, in the matching structure formed by the groove and the protrusion, the longitudinal section of the groove may be rectangular, and the longitudinal section of the matching protrusion is rectangular. The longitudinal section of the groove is parallel to the concave direction of the groove. Of course, the longitudinal section of the groove may be circular, square or trapezoidal, and the longitudinal section of the matching protrusion may be circular, square or a trapezoidal. Preferably, after the first wire pull ring 31 is matched and connected to the first catheter section 10, the connection can be reinforced by laser welding or other methods. In this embodiment, an end of the first pull wire ring 31 is provided with a first pull wire ring groove 312, and the first catheter section 10 is provided with a matching first catheter section protrusion 101.

Similarly, as shown in Fig. 4, an end of the second pull wire ring 41 is provided with a groove/protrusion, and the portion of the second catheter section 20 connected to the second pull wire ring 41 is provided with a protrusion/groove. The second wire pull ring 41 and the distal end of the second catheter section 20 are connected through the matching of the groove and the protrusion. For details, please refer to the above-mentioned description of the connection between the first pull wire ring 31 and the first catheter section 10, which will not be described again here. The portion of the first catheter section 10 in connection with the second pull wire ring 41 is provided with a protrusion/groove, so that the second pull wire ring 41 and the proximal end of the first catheter section 10 are connected through the matching of the groove and the protrusion, thereby achieving connection between the first catheter section 10, the second wire pull ring 41 and the second catheter section 20. The angles at which the first catheter section 10 and the second catheter section 20 can bend can be set or fixed in advance according to the requirements for delivery. In this embodiment, the distal end of the second pull wire ring 41 is provided with a second pull wire ring protrusion 412, and the first catheter section 10 is provided with a first catheter section groove 102 at a matching position. The proximal end of the second pull wire ring 41 is provided with a second pull wire ring groove 413, and the second catheter section 20 is provided with a second catheter section protrusion 201 at a matching position.

Preferably, the connection between the first pull wire ring 31 and the first catheter section 10, the connection between the second pull wire ring 41 and the first catheter section 10, or the connection between the second pull wire ring 41 and the second catheter section 20, is formed by matching exclusive connection structures. In other words, they are assembled and matched in an exclusive way, so that the first pull wire ring 31, the first catheter section 10, the second pull wire ring 41 and the second catheter section 20 can be assembled in an orderly manner, avoiding misalignment of the components and misoperation during assembly. For example, the connection between the first pull wire ring 31 and the first catheter section 10 is exclusive. The shape and size of the groove/protrusion of the first pull wire ring 31 match with the shape and size of the protrusion/groove of the first catheter section 10, but they do not match the shapes and sizes of other grooves and protrusions, so that the first wire pull ring 31 cannot be connected to the second catheter section 20. Likewise, the connection between the proximal end of the second pull wire ring 41 and the distal end of the second catheter section 20 is exclusive, so as to prevent the proximal end of the second pull wire ring 41 from connecting to the first catheter section 10. Similarly, the connection between the distal end of the second pull wire ring 41 and the proximal end of the first catheter section 10 is exclusive, so as to prevent the distal end of the second pull wire ring 41 from connecting to the second catheter section 20.

Furthermore, an exclusive connection structure is provided on the first wire pull ring 31 along the circumferential direction thereof to connect to the first catheter section 10, and a further exclusive connection structure is provided on the second wire pull ring 41 along the circumferential direction thereof to connect to the first catheter section 10 or the second catheter section 20. For example, the first wire pull ring 31 and the first catheter section 10 have at least two matching structures each formed by a groove and a protrusion. Each matching structure formed by a groove and a protrusion is different in shape and size, so that the first pull wire ring 31 and the first catheter section 10 can be assembled in an exclusive manner. Similarly, the second pull wire ring 41 and the second catheter section 20 have three matching structures each formed by a groove and a protrusion. The three matching structures may be evenly arranged along the circumferential direction of the second pull wire ring 41. At least one of the matching structures is different from the other two in shape and size. For example, at least one of the matching structures is smaller in width than the other two. A narrow protrusion can only be inserted into a narrow groove, and a wide protrusion can only be inserted into a wide groove, thereby achieving exclusive assembly between the second pull wire ring 41 and the second catheter section 20, ensuring that they are assembled correctly in position. Similarly, the second pull wire ring 41 and the first catheter section 10 also have an exclusive matching structure, which will not be described again.

Preferably, as shown in Fig. 5, the proximal or distal end of the first catheter section 10 is provided with a positioning mark 13. The positioning mark 13 is, for example, a positioning hole, configured to determine the circumferential position of the first catheter section 10. For example, the first catheter section 10 is provided with positioning holes at both the proximal end and the distal end. Furthermore, two positioning holes are formed at the proximal end. The two positioning holes are arranged opposite each other to respectively indicate the back and the opposite of the back of the first catheter section 10, so as for assisting positioning during the assembling process. Similarly, two positioning holes are formed at the distal end. The two positioning holes are arranged opposite each other to respectively indicate the back and the opposite of the back of the first catheter section 10, so as for assisting positioning during the assembling process.

Preferably, the first pull wire ring 31 has a pull wire clamping groove, which is implemented here as a first pull wire clamping groove 311, and the first pull wire 32 is configured to be clamped in the pull wire clamping groove, so that the first pull wire 32 is connected to the first pull wire ring 31. Similarly, after the first pull wire 32 is matched and connected to the pull wire clamping groove, the connection can be reinforced through laser welding or other methods. Preferably, there are two first pull wires 32. Accordingly, the first pull wire ring 31 has two first pull wire clamping grooves 311, and each first pull wire 32 is respectively clamped in one of the first pull wire clamping grooves 311. Similarly, the second pull wire ring 41 has a pull wire clamping groove, which is implemented as for example a second pull wire clamping groove 411, and the second pull wire 42 is configured to be clamped in the pull wire clamping groove, so that the second pull wire 42 is connected to the second pull wire ring 41. The pull wire clamping groove is in clamping connection with the pull wire, and can also position the pull wire. Preferably, the connection between the pull wire clamping groove and the pull wire can be further fixed by welding. Similarly, after the first pull wire 32 is matched and connected to the pull wire clamping groove, the connection can be reinforced through laser welding or other methods. The first wire pull ring 31 or the second wire pull ring 41 can be prepared by, but is not limited to, laser cutting, machining, pouring and lost-wax sintering.

Preferably, as shown in Fig. 2, the delivery catheter further includes a lining tube 50. The lining tube 50 is disposed inside the first catheter section 10 and the second catheter section 20. The first pull wire 32 is disposed between the first catheter section 10, the second catheter section 20 and the lining tube 50. The second pull wire 42 is disposed between the second catheter section 20 and the lining tube 50. The lining tube 50 is preferably arranged in close contact with the first catheter section 10 and the second catheter section 20, so that the first pull wire 32 and the second pull wire 42 can be squeezed into the gap therebetween, thereby preventing the first pull wire 32 and the second pull wire 42 from being misplaced. Furthermore, a pull wire cavity 60 is provided in the gap between the lining tube 50 and the first and second catheter sections 10, 20. The pull wire cavity 60 is, for example, configured to provide a space for placement of the first and second pull wires 32, 42. The first pull wire 32 is implemented as, for example, two pull wires, and the two pull wires are located on opposite sides of the first catheter section 10. The second pull wire 42 is implemented as one pull wire which is for example located at a position angled 90° to the two first pull wires 32. Accordingly, the pull wire cavity 60 is implemented as three pull wire cavities. The pull wires are, for example, strong pull wires. All the pull wires extend from their proximal end to their distal ends and emerge from the distal end of the delivery catheter. When the two first pull wires 32 are pulled, the bending of the first catheter section 10 is achieved. When the second pull wire 42 is pulled, the bending of the second catheter section 20 is achieved. The use of two wire pull wire rings can ensure that the first catheter section 10 and the second catheter section 20 are bent in specified bending directions. Furthermore, by adjusting the first catheter section 10 and the second catheter section 20, different bending angles and bending radii can be achieved without interference.

Preferably, the body of the first pull wire ring 31 or the second pull wire ring 41 has a hole, in which the lining tube 50 can expand to increase the friction resistance between the lining tube 50 and the first pull wire ring or the second pull wire ring 41, thereby improving the connection strength and the production quality of the delivery catheter. For example, three round holes 313 are distributed evenly on the body of the first pull wire ring 31 along the circumferential direction thereof. Of course, the hole may be square, triangular, etc., and the hole may be implemented as one or more. In other embodiments, the second wire pull ring 41 may also be provided with a hole.

Preferably, the delivery catheter further includes a covering tube 70, which is disposed outside the first catheter section 10 and the second catheter section 20, thereby reducing the frictional resistance between the delivery catheter and the blood vessel.

More preferably, the delivery catheter further includes a braided wire 80, which is disposed at the proximal end of the second catheter section 20. The braided wire 80 and the second catheter section 20 are connected together by, for example, laser welding.

The use of the delivery catheter in this embodiment in two different scenarios will be introduced below with reference to Figs. 7 and 8.

Scenario 1: The delivery catheter can be used for, but is not limited to, the delivery and deployment of the mitral valve prosthesis via a transseptal approach. The delivery catheter passes through the femoral vein and the inferior vena cava into the heart. It passes through the interatrial septum and then turns toward the mitral valve. Then the first catheter section 10 forms a first bending 1 in the left atrium that is coaxial with the axis of the mitral valve. At the same time, the second catheter section 20 at the tail end of the ring can be bent toward the mitral valve to form a second bending 2, which is used to adjust the relative position of the first bending 1 with the axis of the mitral valve. As shown in Figs. 7 and 8, which illustrate the bending of the delivery catheter, a first bending 1 of 270° can be formed at the distal end of the catheter, and a second bending 2 of 45° can be formed at the end of the first bending 1. The distal end of the first bending 1 can reach the anterior or posterior commissure of the mitral valve to prepare for deployment of the cardiac implant.

Scenario 2: The delivery catheter may be used for, but is not limited to, the delivery and deployment of the mitral valve prosthesis via a transfemoral approach. The delivery catheter passes through the femoral artery and the aortic arch into the heart. It passes through the aortic valve and then turns toward the mitral valve. Then the first catheter section 10 forms a first bending 1 in the ventricle that is coaxial with the axis of the mitral valve. At the same time, the second catheter section 20 at the tail end of the ring can be bent toward the mitral valve to form a second bending 2, which is used to adjust the relative position of the first bending 1 with the axis of the mitral valve. The distal end of the first bending 1 can reach below the mitral valve leaflets and wrap around the chordae tendineae to prepare for deployment of the implant.

The delivery catheter according to this embodiment provides a catheter that can be controllably bent in different sections and directions to match the internal dimensions of the ventricle and provide transmission and release for the valve anchoring ring.

In conclusion, in a delivery catheter provided by the present invention, the delivery catheter includes: a first catheter section, a second catheter section, a first pull wire assembly and a second pull wire assembly; the first pull wire assembly includes a first pull wire and a first pull wire ring, the first pull wire ring is provided at the distal end of the first catheter section, the first pull wire is connected to the first pull wire ring and extends toward the proximal end of the first catheter section; when the first pull wire is pulled, the first pull wire ring drives the first catheter section to bend; the second pull wire assembly includes a second pull wire and a second pull wire ring, and the second pull wire ring is disposed at the distal end of the second catheter section and is connected to the proximal end of the first catheter section; the second pull wire is connected to the second pull wire ring and extends toward the proximal end of the second catheter section; when the second pull wire is pulled, the second pull wire ring drives the second catheter section to bend; the angle at which the first catheter section can bend is greater than the angle at which the second catheter section can bend. With this arrangement, the delivery catheter can have two bendable sections with different characteristics. The first pull wire assembly is configured to control the bending of the first catheter section, and the second pull wire assembly is configured to control the bending of the second catheter section, so that the bending of the first catheter section and the second catheter section is controllable. It can realize a segmented bending structure with different bending angles, achieving different bending radii, bending angles and bending directions of the catheter during delivery. It can also realize a multipoint control of bending, which facilitates the implant to pass through the blood vessel smoothly and be delivered to the target location, and thus the problems of little difference in terms of the performance of the bendable section, such as bending angles, and having insufficient bending are solved.

In addition, it should be recognized that although the present invention has been disclosed above in preferred embodiments, the above embodiments are not intended to limit the present invention. For any person familiar with the art, without departing from the scope of the technical solution of the present invention, they can use the technical content disclosed above to make many possible changes and modifications to the technical solution of the present invention, or modify it into equivalent embodiment with equivalent changes. Therefore, any simple modifications, equivalent changes and modifications made to the above embodiments based on the technical essence of the present invention without departing from the content of the technical solution of the present invention still fall within the scope of protection of the technical solution of the present invention.

## Claims

1. A delivery catheter, comprising a first catheter section, a second catheter section, a first pull wire assembly and a second pull wire assembly; wherein
the first pull wire assembly comprises a first pull wire and a first pull wire ring, wherein the first pull wire ring is disposed at a distal end of the first catheter section, the first pull wire is connected to the first pull wire ring and extends toward a proximal end of the first catheter section; when the first pull wire is pulled, the first pull wire ring drives the first catheter section to bend;
the second pull wire assembly comprises a second pull wire and a second pull wire ring, wherein the second pull wire ring is disposed at a distal end of the second catheter section and connected to the proximal end of the first catheter section, the second pull wire is connected to the second pull wire ring and extends toward a proximal end of the second catheter section; when the second pull wire is pulled, the second pull wire ring drives the second catheter section to bend;
an angle at which the first catheter section can bend is greater than an angle at which the second catheter section can bend.

2. The delivery catheter according to claim 1, wherein the first catheter section comprises a hypotube section, and the second catheter section comprises a serpentine section.

3. The delivery catheter according to claim 1, wherein the first catheter section comprises a bendable section and a transition section, and wherein the bendable section is connected to the transition section and is located distally to the transition section, and an angle at which the bendable section can bend is greater than an angle at which the transition section can bend.

4. The delivery catheter according to claim 1, wherein an end of the first pull wire ring is provided with a groove, and a portion of the first catheter section in connection with the first pull wire ring is provided with a protrusion, or an end of the first pull wire ring is provided with a protrusion, and a portion of the first catheter section in connection with the first pull wire ring is provided with a groove;
wherein the first wire pull ring and the distal end of the first catheter section are connected through matching of the groove and the protrusion.

5. The delivery catheter according to claim 1, wherein a proximal end of the second pull wire ring is provided with a groove, and a portion of the second catheter section in connection with the second pull wire ring is provided with a protrusion, or a proximal end of the second pull wire ring is provided with a protrusion, and a portion of the second catheter section in connection with the second pull wire ring is provided with a groove;
wherein the second wire pull ring and the distal end of the second catheter section are connected through matching of the groove and the protrusion.

6. The delivery catheter according to claim 1, wherein a distal end of the second pull wire ring is provided with a groove, and a portion of the first catheter section in connection with the second pull wire ring is provided with a protrusion, or a distal end of the second pull wire ring is provided with a protrusion, and a portion of the first catheter section in connection with the second pull wire ring is provided with a groove;
wherein the second wire pull ring and the proximal end of the first catheter section are connected through matching of the groove and the protrusion.

7. The delivery catheter according to claim 1, wherein a connection between the first pull wire ring and the first catheter section, a connection between the second pull wire ring and the first catheter section, or a connection between the second wire pull ring and the second catheter section, is formed by matching exclusive connection structures.

8. The delivery catheter according to claim 1, wherein an exclusive connection structure is provided on the first pull wire ring along a circumferential direction thereof to connect to the first catheter section, and a further exclusive connection structure is provided on the second pull wire along a circumferential direction thereof to connected to the first catheter section or the second catheter section.

9. The delivery catheter according to claim 1, wherein a positioning mark is provided at the proximal end or the distal end of the first catheter section, and the positioning mark is configured to determine a circumferential position of the first catheter section.

10. The delivery catheter according to claim 1, wherein the first pull wire ring or the second pull wire ring has a pull wire clamping groove in which the first pull wire or the second pull wire can be clamped.

11. The delivery catheter according to claim 1, wherein the delivery catheter further comprises a lining tube disposed inside the first catheter section and the second catheter section, and the first pull wire is disposed between the first catheter section, the second catheter section and the lining tube, and the second pull wire is disposed between the second catheter section and the lining tube.

12. The delivery catheter according to claim 11, wherein a body of the first pull wire ring or the second pull wire ring has a hole into which the lining tube can expand outward so that a friction resistance between the lining tube and the first pull wire ring or the second pull wire ring is increased.

13. The delivery catheter according to claim 1, further comprising a covering tube disposed outside the first catheter section and the second catheter section.
